Europäisches Patentamt

European Patent Office

Office européen des brevets

(11) Publication number: **0 479 448 A2**

# EUROPEAN PATENT APPLICATION

(21) Application number: **91308492.7**

(22) Date of filing: **18.09.91**

(51) Int. Cl.5: **G01N 33/551, B03C 1/00**

(30) Priority: **02.10.90 US 591649**

(43) Date of publication of application:
**08.04.92 Bulletin 92/15**

(84) Designated Contracting States:
**DE FR GB IT**

(71) Applicant: **BECKMAN INSTRUMENTS, INC.**
**2500 Harbor Boulevard Box 3100**
**Fullerton California 92634-3100(US)**

(72) Inventor: **Pfost, R. Fred**
**1090 Eastwood Ct.**
**Los Altos, California 94022(US)**
Inventor: **Avdalovic, Nebojsa**
**10169 N. Blaney Avenue**
**Cupertino, California 95014(US)**

(74) Representative: **Ede, Eric et al**
**Fitzpatricks 4 West Regent Street**
**Glasgow G2 1RS(GB)**

(54) **Magnetic separation device.**

(57) A magnetic separation device (30) adapted for automatic activation using an automated laboratory workstation (10). In the described embodiment, the device comprises a magnetic plate (70) having an aperture for receiving a receptacle (73) containing magnetizable beads (75). The magnetic plate (70) is raised with respect to the receptacle (73) by a lever mechanism to a level to engage the plate so as to hold the beads (75) against the inside vertical wall of the receptacle (73). A release mechanism is provided to lower the plate to disengage the magnetic plate (70). The lever mechanism and the release mechanism can be activated by the automated laboratory workstation (10).

FIG. 2

# BACKGROUND OF THE INVENTION

## 1. Field of the Invention

The present invention relates to conducting reactions of biomolecules utilizing a solid phase to immobilize the biomolecules of interest and more particularly to a device for isolating the biomolecules from suspension utilizing a magnetic solid phase.

## 2. Description of Related Art

Solid-Phase methods have proven to be very useful for the separation, synthesis and diagnostic detection of biomolecules. Basically, the method involves the use of a solid support surface for reaction of biomolecules to take place and for binding the resultant biomolecules to thereby isolate the biomolecules of interest. The solid phase approach produces reproducible reactions with high yields, and allows solutions to be changed rapidly. The isolated biomolecules can be easily separated from the reaction solution. The solid phase may consist of walls of test tubes or microtiter wells, or polymer particles.

Magnetic particles can also be used as a solid support for the separation of biomolecules in solution without the need for centrifugation or filtration. There are two primary components in magnetic separations: (a) particles which have magnetic or magnetizable property and will serve as a solid phase to remove biomolecules of interest out of solution, and (b) a suitable source of magnetic field for immobilizing the particles. Typically, particles in the form of beads are used as the solid phase. The beads used are derivatized magnetic or magnetizable particles which are treated with a binding material that can bind and remove from solution various kinds of biomolecules depending on the chemical nature of the binding material. For example, antigen or antibody coated beads have been used for immunoassays. If the magnetic beads are non-porous, adsorption and desorption of biomolecules occur at the surface, providing reaction kinetics similar to those found in free solution. The biomolecules of interest can be separated from the solution by subsequently separating the beads from the solution and extracting the bound biomolecules from the beads. Magnetic separations have been found to be useful for procedures such as immunoassays, cell separations, nucleic acid assays, and purifications of biological material.

In the past, several techniques have been practiced to apply magnetic field to immobilize the beads. They generally involve one of two approaches. In one approach, the magnet is placed against the outside vertical wall of the solution container, e.g. a test tube, to hold the beads against the inside vertical wall of the container. The reagent solution is then poured or otherwise siphoned out of the container while the beads are immobilized by the magnet. Subsequently, the magnet may be removed from the container and the beads can be collected. More typically, the beads are left in the container while a wash solution is introduced to wash the beads and container to remove residual reagent. The wash solution can be removed by aspiration while applying the magnet to immobilize the beads. A new reagent is subsequently introduced for a second reaction with the biomolecules bound on the beads. This reaction may involve extraction of the biomolecules from the beads.

In another approach, the magnet is placed above an inverted container which is placed above the opening of the container containing the solution and the beads. The magnetic force lifts the beads out of solution and holds them in the inverted container. The magnet and inverted container with beads are then placed above another container containing appropriate reagent or wash solution and the magnet is removed to release the beads into this container. This approach has been practiced by Organon Teknika Corporation in their "Magnetic Transfer Device".

The steps involved in the above approaches so far have been carried out manually. For those procedures which require repeated washing and addition of reagents in sequence, it becomes laborious to do so manually. One can appreciate the laborious task of manually carrying out the steps for multiple containers such as multi-well titer plates. Furthermore, it was rather stressful for the laboratory personnel to have to position the pipette tip accurately to avoid hitting the beads when inserting the pipette tip into the small opening of a receptacle, e.g. 96-well titer plate.

# SUMMARY OF THE INVENTION

The present invention is directed to a magnetic separation device which carries out magnetic separation in an automated sequence. In one embodiment of the present invention, the device comprises a magnetic separation module and a robotic assembly. The module is designed to allow the robotic assembly to engage the magnet with respect to the receptacle to immobilize the magnetizable beads.

In the described embodiment, the module comprises a frame on which lever arms are supported for actuating a magnet plate. The magnet plate is lowered or raised with respect to the module by the lever arms. In the raised (engaged) position, the bottom of each well of the titer plate protrudes through an aperture of the magnet plate. The magnetic field of the magnet plate is perpendicular to the plate. The magnetizable beads are held by the magnetic field against the inside vertical wall of the wells. In the lowered (disengaged) position, the magnet plate is at a sufficient distance below the titer plate such that magnetic influence on the beads is substantially reduced to allow the beads to fall to the bottom of the wells.

The lever arms are actuated by a push rod located on the frame. The push rod can be activated by means of a robotic assembly in an automated laboratory workstation of which the module is a part. The workstation is program controlled to transfer fluid to and from the receptacle and activate the magnetic separation module automatically in accordance with a desired sequence of steps.

BRIEF DESCRIPTION OF THE DRAWINGS

Fig. 1 is a perspective view of an automated workstation equipped with a magnetic separation module in accordance with one embodiment of the present invention.

Fig. 2 is an assembly drawing showing the various components of the magnetic separation module in accordance with one embodiment of the present invention.

Fig. 3 is a plan view of the lever arm assembly in the magnetic separation module.

Fig. 4 is a sectional view taken from line 4-4 in Fig. 3.

Fig. 5 is an end view of the lever arm assembly shown in Fig. 3.

Fig. 6 is another end view of the lever arm assembly shown in Fig. 3.

Fig. 7 is a plan view of the frame of the magnetic separation module.

Fig. 8 is a side view of the frame shown in Fig. 7.

Fig. 9 is an end view of the frame shown in Fig. 7.

Fig. 10 is a sectional view taken along line 10-10 in Fig. 7.

Fig. 11 is a sectional view taken along line 11-11 in Fig. 7.

Fig. 12 is a sectional view taken along line 12-12 in Fig. 7 which shows the push rod assembly installed.

Fig. 13 is a sectional view taken along line 13-13 in Fig. 7.

Fig. 14A illustrates the lowered (disengaged) configuration of the magnetic separation module. Fig. 14B is a sectional view taken along line 14B-14B in Fig. 14A.

Fig. 15A illustrates the raised (engaged) configuration of the magnetic separation module. Fig. 15B is a sectional view taken along line 15B-15B in Fig. 15A.

Fig. 16A illustrates the position of the magnet plate in the disengaged position with respect to the well of a titer plate. Fig. 16B illustrates the engaged position of the magnet plate with respect to the well of the titer plate. Fig. 16C is a top view of the well showing the configuration of the magnetizable beads in the engaged position of the magnet plate.

Fig. 17A shows the push rod; Fig. 17B is a bottom axial view of the push rod.

Fig. 18A shows the wedge for locking the push rod; Fig. 18B is a top axial view of the push rod; Fig. 18C is a bottom axial view of the push rod.

DESCRIPTION OF THE ILLUSTRATED EMBODIMENT

The following description is of the best presently contemplated mode of carrying out the invention. This description is made for the purpose of illustrating the general principles of the invention and should not be taken in a limiting sense. The scope of the invention is best determined by reference to the appended claims.

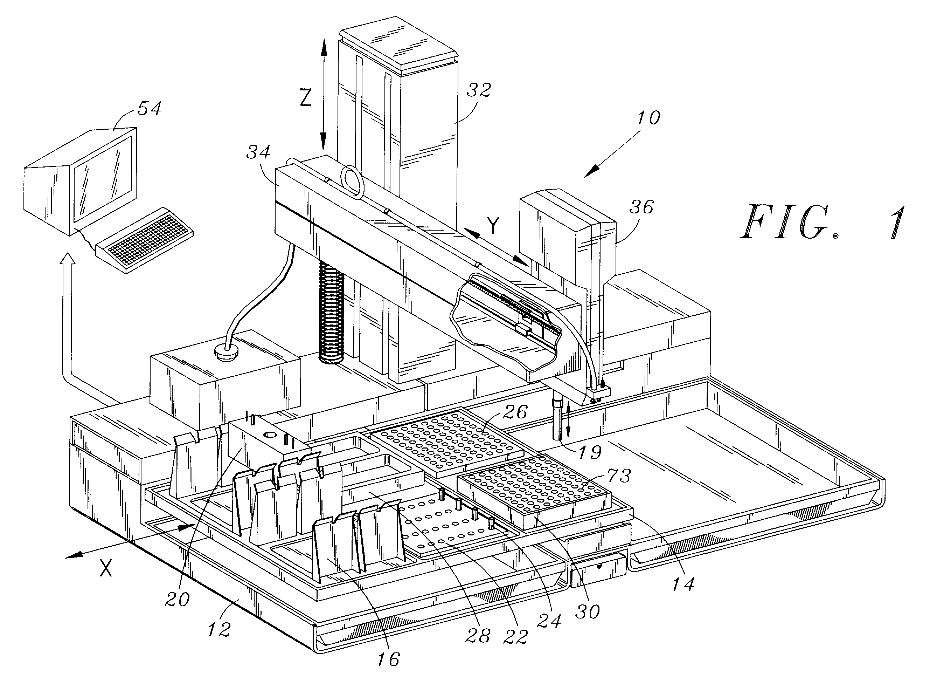

Prior to a detailed description of the magnetic separation module, the overall configuration of the robotic assembly and the magnetic separation module in accordance with one embodiment of the present invention will be described. One embodiment of the magnetic separation module has been developed by Beckman Instruments, Inc. in connection with their BIOMEK® 1000 automated laboratory workstation. Referring to Fig. 1, a perspective view of an automated laboratory workstation 10 similar to the BIOMEK® 1000 is shown. This workstation has been described in detail in copending U.S. patent application Serial No. 07/383,299 which is commonly assigned to the assignee of the present invention and is incorporated by reference. For purposes of discussion of the present invention herein, only the relevant components of the automated laboratory workstation 10 shown in Fig. 1 will be described.

The workstation 10 comprises a base 12 on which a tablet 14 is moved horizontally (arrow X) by stepping motors (not shown). The tablet 14 supports items including: a number of tool stands 16 for holding various tools, e.g. pipette 20; a tray 22 for holding pipette tips 24 to be fitted to the end of the pipette; multi-well titer plate 26; a multi-reservoir tray 28 for holding reagent solutions; and other labwares required for carrying out a sequence of laboratory operations associated with for example magnetic separation, DNA sequencing, etc. Also supported on the tablet 14 is the magnetic separation module 30 which will be described in detail below. A tower 32 vertically extends from the base 12. A horizontally support arm 34 can be moved vertically (arrow Z) along the tower 32. A pod 36 is supported at the end of the arm 34. The pod 36 is designed to pick up and manipulate the tools. The pod 36 comprises a plunger 19 which is used to operate the pipette, for example. The pod 36 is movable horizontally along the arm 34 in a transverse direction (arrow Y) with respect to the tablet horizontal movement (arrow X). It can be seen that through the combined vertical (Z), horizontal (X) and transverse (Y) motions of the arm 34, tablet 14 and pod 36, respectively, a series of laboratory operations can be sequentially performed, for example pipetting a desired quantity of reagent from the reservoir 28 into the multi-well titer plate in the magnetic separation module 30, aspirate spent reagent from the magnetic separation module 30, washing the titer plate 73 in the module 30, etc. The movements of the various robot components are actuated by stepper motors (not shown) and lead screws (not shown in detail) controlled by a mini-computer 54. Once the computer 54 has been programmed to control the desired sequence of operations, user intervention would no longer be required until the programmed operations have been completed. One can appreciate that a large number of repetitive operations can be carried out effortlessly by the automated laboratory workstation 10.

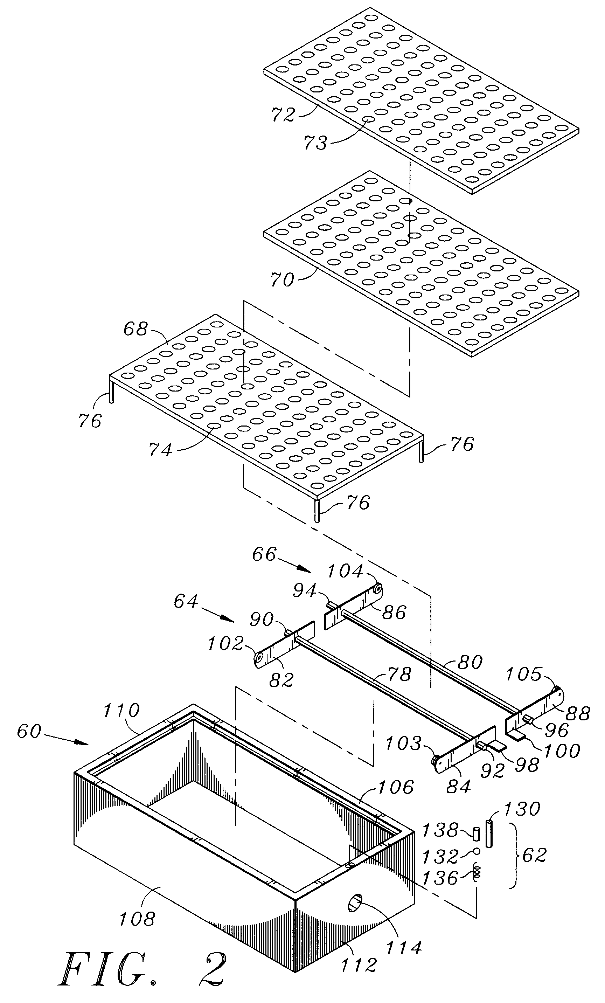

The magnetic separation module will now be described. Fig. 2 shows the assembly of one embodiment of the magnetic separation module 30. While the illustrated embodiment is designed for accommodating a 96-well titer plate as the reagent and magnetizable bead receptacle, it is apparent from the discussion hereinbelow that the module can be easily modified to accommodate other types of receptacles such as test tubes. The components of the module 30 comprises: frame 60, push rod assembly 62, lever assemblies 64 and 66, aperture plate 68, magnet plate 70 and 96-well titer plate 72.

The aperture plate 68 is made of non-magnetizable material approximately 0.125 inch thick. There is an array of 8x12 0.25 inch diameter circular apertures 74 at approximately 9mm center-to-center spacing. These apertures 74 conform to the configuration of the underside of the 96-well titer 72 plate such that the bottom of each well 73 can be received through each aperture 74 when the aperture plate is held against the bottom of the titer plate 72 (see Figs. 15 and 16 and the discussion in reference thereto). Guide pins 76 are provided and extends downwardly at the corners of the aperture plate 68.

The magnet plate 70 is permanent magnet material approximately 0.06 inch thick. It is magnetized perpendicular to its plane, i.e. the magnetic north and south pole lie respectively on the top and bottom planar surfaces of the material (see Fig. 16). The material should have a high coercivity in order to maintain a high magnetic field. It has been found that a rubber impregnated with Samarium iron particles is a suitable material. The magnet plate 70 is perforated much like the aperture plate 68. The magnet plate 70 is supported on the top surface of the aperture plate 68. It is understood that the magnet plate 68 can be omitted by making the aperture plate 74 of a permanent magnet material. Alternatively, a thin torrous made of rare earth magnetic material could be secured to the aperture plate 68 at each aperture location to provide the required magnetic field. The magnetic field could also be obtained electromagnetically by use of a coil of wire at each aperture location and electrically energizing the coil.

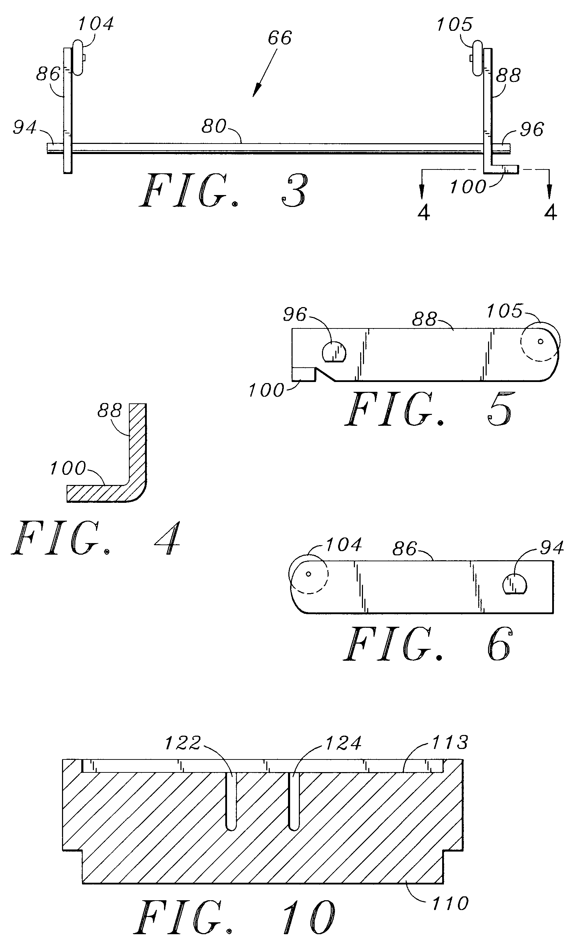

Referring to Figs. 2-6, each lever assembly (64, 66 Fig. 2) (lever assembly 64 is a mirror image of lever assembly 66) comprises a rod (78, 80) and a lever arm (82, 84, 86, 88) rigidly attached to each end of the rod. The ends (90, 92, 94, 96) of the rod extend slightly beyond the lever arms. The lever arm (84, 88) in each assembly has a tab (98, 100) extending from the plane of the arm. At the end of each arm (82, 84, 86, 88) there is provided a roller bearing (102, 103, 104, 105) with axis parallel to the rod (78, 80).

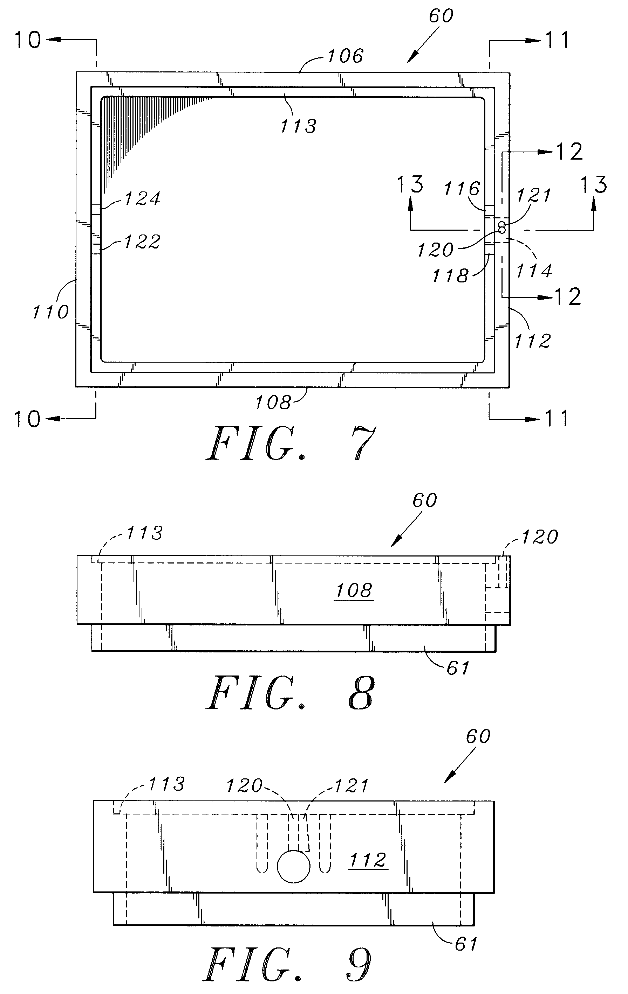

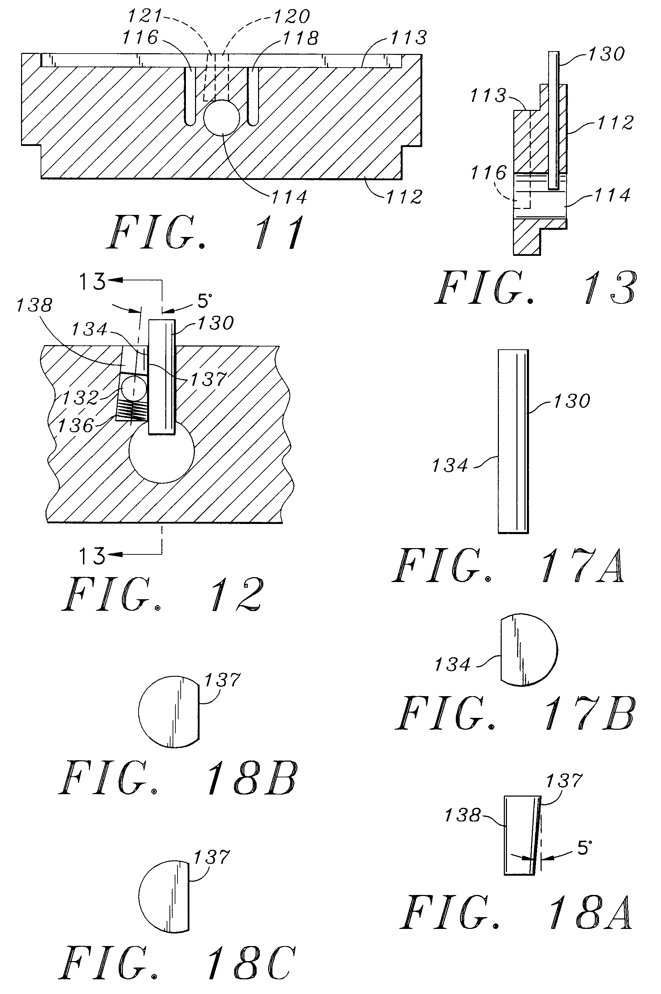

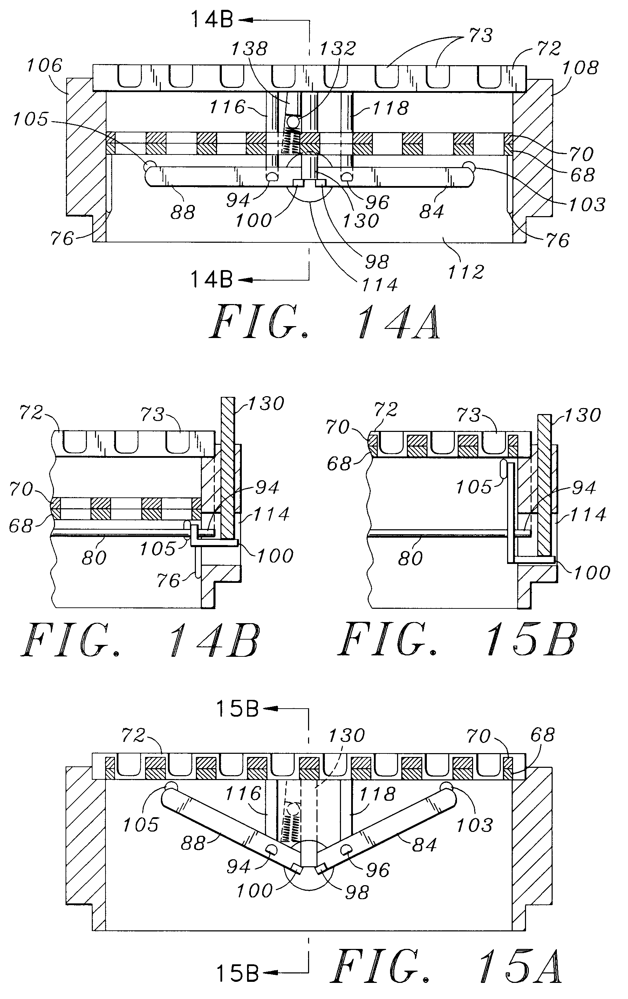

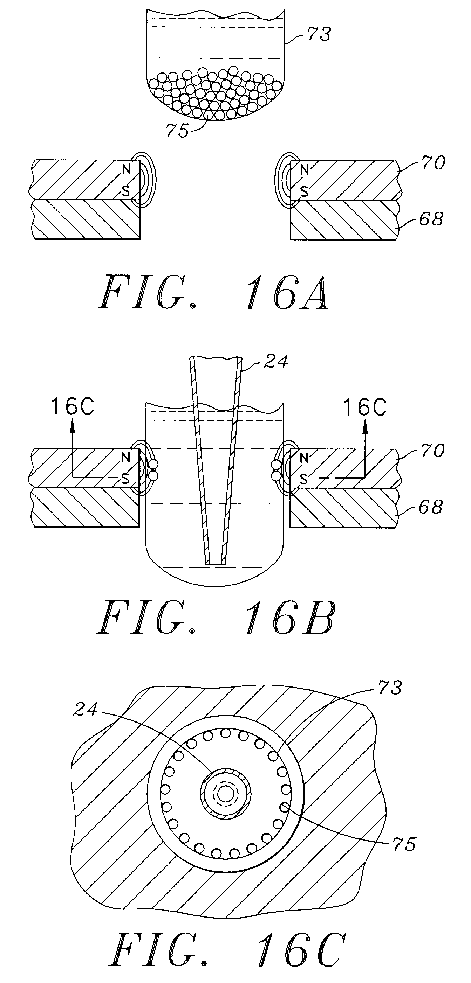

Referring to Figs. 7-13, the frame 60 is rectangular with opposing sidewalls (106, 108) and endwalls (110, 112) defining a central space for receiving the aperture plate 68 and the magnet plate 70. The 96-well titer plate 72 is to be supported by the walls (106, 108, 110, 112) along the top edge of each wall by a shoulder 113 which is for aligning the 96-well titer plate 72 with the frame 60 by restraining sideway movement of the titer plate 72 relative to the frame 60 (see for example Fig. 14). The end wall 112 of the frame has a hole 114. Adjacent the hole 114 are two slots (116, 118). Perpendicular to the axis of the hole 114 is a longitudinal hole 120 for slidably receiving the push rod 130. Another longitudinal hole 121 is provided at a 5° angle to the hole 120 for receiving the wedge 138, ball 132 and spring 136 (see Fig. 12). The push rod 130, spring 136, ball 132 and wedge together make up the push rod assembly 62. Referring to Fig. 17, the cylindrical push rod has a flat 134 machined in the axial direction. Referring to Fig. 18, the wedge 138 has a flat 137 machined at a 5° angle to the axis of the cylindrical wedge 138. When assembled on the frame 60, the flat 137 of the wedge faces the flat 134 of the push rod as shown in Fig. 12. The ball 132 and wedge 138 are biased by the spring 136 to lock the push rod 130 in place. The wedge 138 is also used to unlock the push rod 130. These will be explained in connection with the operation of the module. The frame end wall 110 also has two slots (122, 124) which are directly opposing the slots (116, 118). The height of the walls (106, 108, 110, 112) are sized such that there is sufficient room for the aperture plate 68 to be raised and lowered to respectively engage and disengage the magnet plate 70 with respect to the titer plate 72. This will become clear when the operation of the module 30 is explained below. The frame 60 should preferably be made of non-magnetic material in order to avoid interfering with the magnetic field of the magnet plate 70.

It is apparent that should receptacles other than 96-well titer plates be used with the module, different aperture plates can be shaped to conform to the configuration of the receptacles. The frame and magnet plate can be shaped accordingly.

Referring to Figs. 14 and 15 which is the sectional view looking from within the frame at the end wall 112, the assembly and operation of the module 30 are more clearly shown. The lever assemblies (64, 66 Fig. 2) are installed in the middle of the central space defined by the walls (106, 108, 110, 112). Specifically, the protruding ends (90, 92, 94, 96) of the rods (78, 80) are loosely supported by the bottom of the slots (116, 118, 122, 124). The tabs (98, 100) extend into the hole 114 and are positioned under the lower end of the push rod. Figs. 14A and B show the lowered (disengaged) configuration of the aperture plate 68. In this configuration, the aperture plate 68 is positioned at a sufficient distance below the titer plate 72 such that magnetic influence on the beads in the wells of titer plate 72 are substantially reduced. The aperture plate 68 rest on the roller bearings (102, 103, 104, 105) on the ends of the lever arms (82, 84, 86 and 88). The tabs (96, 98) either touch the lower tip of the push rod 130 or rest slightly below the lower tip.

Figs. 15A and B show the raised (engaged) configuration of the aperture plate. In this configuration, the aperture plate 68 is raised towards the bottom of the titer plate 72 and the bottom of each well 73 protrudes through an aperture 74 of the aperture plate 68. Thus each aperture of the magnet plate 70 surrounds a well 73 of the titer plate 72 at a location above the well bottom (see Fig. 16B). The aperture plate 68 is raised by forcing downward on the push rod 130 to cause the lever arms (82, 84, 86, 88) to raise their ends which have the roller bearings (102, 103, 104, 105). Specifically, the lever arms pivot on the protruding ends (90, 92, 94, 96) of the rods (78, 80) against the bottoms of the slots (116, 118, 122, 124) under the action of the push rod 130. As the push rod 130 is forced downwards, frictional contact between the ball 132 and the flat 134 of the push rod 130 moves the ball 132 slightly downwards against the bias of the spring 136 thereby releasing the locking action of the ball 132. Upon release of the downward force on the push rod 130, the ball 132 under the bias of the spring 136 presses on the flat 134 of the push rod 130 to lock the push rod 130 in place to prevent the push rod 130 from sliding upwards. It has been found that the 5° incline (locking angle) of the force of the ball 132 is sufficient to lock the push rod 130 against the weight of the aperture plate 68 on the lever arms (82, 84, 86, 88). The wedge 138 is only used to unlock the ball 132 by pressing it down and consequently away from the flat 134. The four corner guide pins 76 guide the vertical movement of the aperture plate 68 within the frame 60 so as to prevent tilting of the aperture plate 68 which otherwise would spill the contents of the wells and may lead to the aperture plate 68 locking against the walls of the frame.

To lower the aperture plate 68, the wedge 138 is pushed downwards to disengage the ball 132 from the flat 134 of the push rod 130. The tabs (98, 100) on the lever arms (84, 88) pushes the push rod upwards as the lever arms are pivoted under the weight of the aperture plate 68 and the magnet plate 70. Owing to the location of the fulcrum of the lever arms, the combined weight is adequate for lowering the aperture plate 68 without the need for additional force.

Referring back to Fig. 1, the operation of the magnetic separation module 30 in conjunction with the automated workstation 10 will be explained. Fig. 1 shows the module supported on the tablet 14 at a location normally designed for supporting a 96-well titer plate. It is noted that the base 61 of the module 30 is sized to have approximately the same planar area as a 96-well titer plate so that the module can be located at any one of the locations designed for positioning a titer plate, i.e. the module 30 can be interchanged with a titer plate without modification of the tablet 14.

Initially, beads 75 that are magnetizable and having a suitable coating of reactive material is deposited into the wells 73 of the titer plate 72 (see Fig. 16A). As an example for illustration purposes, workstation 10 is programmed to do the following tasks. The appropriate tools, e.g. pipettes 18 are picked up for transferring reagent or sample to and from the wells for reaction with the coating on the beads 75. The pod 36 and tablet 14 are moved in transverse directions so as to position the tools, reagent reservoir 28 and magnetic separation module 30 below the pod 36 in sequence in accordance with the steps of the desired procedure. Sufficient time is allowed for the reaction to complete before magnetic separation.

To "activate" the magnetic separation module 30, the pod 36 is positioned with the depending plunger 19 18 above the push rod 130 of the module 30. The pod 36 is lowered and the plunger 19 activated to press downwards to force the push rod 130 to engage the tabs (98, 100) of the lever arms thereby to raise the aperture plate 68 and the magnet plate 70. Upon release of the plunger 19, the ball 132 locks the push rod in place. To "deactivate" the magnetic separation module 30, the plunger 19 is moved over the wedge 138 and is lowered to force down the wedge 138 to allow the push rod 130 to move upwards under the weight of the aperture plate 68 on the lever arms. Since the movements of positioning hardware, i.e. the tablet 14, arm 50 and hand 52, are effected by stepper motors, very small incremental steps may be made to accurately position the plunger 19 with respect to the push rod 130 and wedge 138.

When the magnet plate 70 is raised, the beads 75 in each well 73 of the titer plate 72 are subject to a magnetic field as shown in Fig. 16B. The beads 75 are held against the inside vertical wall of the well 73. If the beads were uniformly held in the wells 73 prior to application of the magnetic field, the beads 75 will likely form a more or less uniform ring along the inside vertical wall of the well 73 (See Fig. 16C). Thus a central clearance is available for allowing a pipette tip 24 to enter the well 73. It is important that the pipette tip 24 be able to reach the bottom of the well 73 in order to be able to completely remove any fluid in the well. With the beads 75 held uniformly around the well 73, there is sufficient central clearance for lowering the pipette tip 24 to reach the bottom of the well 73. In prior art devices in which a magnet is placed against one side of the receptacle, the beads will accumulate on one side of the inside wall of the receptacle so that there may be insufficient central clearance for a pipette tip to be lower into the center of the receptacle. This is especially so if the pipette tip used is thick.

It is noted that the beads can be made of magnetizable ferrous material for magnetic attraction to the permanent magnet plate. It is within the scope of the present invention to make the aperture plate from ferrous material and the beads from permanent magnet material (the magnet plate will not be required in this embodiment).

In view of the foregoing description, one can appreciate that the otherwise laborious steps involved in magnetic separation can be performed automatically and relatively effortlessly using the automated workstation equipped with a magnetic separation module. Repetitive sequence of steps can be performed accurately for an extended period of time. Positioning of the pipette tip into the wells can be performed with accuracy without fear of hitting the beads.

To further automate the process, a side loader (not shown) having a robot arm can be utilized to load and unload a variety of labware onto and from the tablet. It would not be necessary for an operator to be available to position sample containers and pipette tips on the tablet. The magnetic separation module can be loaded onto the tablet only when its use is called for so as to conserve space on the tablet for other labwares needed for a laboratory procedure. An example of a side loader is the BIOMEK® SL designed to complement the BIOMEK® 1000 automated laboratory workstation.

While the invention has been described with respect to the preferred embodiments in accordance therewith, it will be apparent to those skilled in the art that various modifications and improvements may be made without departing from the scope and spirit of the invention. Accordingly, it is to be understood that the invention is not to be limited by the specific illustrated embodiments, but only by the scope of the appended claims.

**Claims**

1. A magnetic separation device (30) comprising:
   a receptacle (73) containing magnetizable beads (75);
   a magnetic plate (70) having an aperture sized to receive the receptacle (73); and
   means for moving the magnetic plate (70) relative to the receptacle (73) so as to receive the receptacle (73) in the aperture, the magnetic field of the magnetic plate (70) causing the beads (75) to be held against the wall of the receptacle (73).

2. An automated magnetic separation device comprising:
   an automated laboratory workstation (10) having robot means for performing robotic operations in accordance with a desired sequence of steps;
   a magnetic separation module (30) comprising:
   a receptacle (73) containing magnetizable beads (75);
   a magnet (70);
   actuating means (19, 36, 98, 100, 130) for moving the magnet (70) relative to the receptacle (73) so as to cause the beads (75) to be held by magnetic field against the wall of the receptacle (73); and
   controlmeans (54) for controlling the robotic means to activate the actuating means.

3. A device according to claim 2 wherein the magnet is in the form of a plate (70) having an aperture sized to receive the receptacle (73).

4. A magnetic separation device (30) comprising:

a receptacle (73) containing magnetizable beads (75); and

magnetic means (70) for imposing a magnetic field around the periphery of the receptacle (73) so as to hold the beads (75) against the wall in a substantially uniform distribution.

FIG. 1

EP 0 479 448 A2

FIG. 2

FIG. 3

FIG. 4

FIG. 5

FIG. 6

FIG. 10

FIG. 7

FIG. 8

FIG. 9

FIG. 11

FIG. 13

FIG. 12

FIG. 17A

FIG. 17B

FIG. 18B

FIG. 18C

FIG. 18A

FIG. 14A

FIG. 14B

FIG. 15B

FIG. 15A

*73*

*75*

*70*

*68*

## FIG. 16A

*24*

16C

16C

*70*

*68*

## FIG. 16B

*73*

*24*

*75*

## FIG. 16C